# EUROPEAN PATENT SPECIFICATION

(11) **EP 2 881 399 B1**
(45) Date of publication and mention of the grant of the patent: **12.09.2018**
(21) Application number: 14197064.0
(22) Date of filing: 09.12.2014
(51) Int. Cl.: C07F 15/02, C07C 337/08, A61P 35/02, A61P 35/00, A61K 31/295

(54) **New thiosemicarbazone chelates having anticancer activity**
Neue Thiosemicarbazonchelate mit Antitumorwirkung
Nouveaux chélates thiosémicarbazones présentant une activité anticancéreuse

(30) Priority: 09.12.2013 TR 201314429
(43) Date of publication of application: 10.06.2015
(73) Proprietor: Istanbul Universitesi Rektorlugu, 34452 Istanbul (TR); Bal Demirci, Tülay, Instanbul (TR); Ulküseven, Bahri, Istanbul (TR); Kuruca, Dürdane Serap, Istanbul (TR)
(72) Inventor: Bal Demirci, Tülay, Istanbul (TR); Ulküseven, Bahri, Istanbul (TR); Kuruca, Dürdane Serap, Istanbul (TR)
(74) Representative: Mutlu, Aydin

(56) References cited:
- TÜLAY BAL ET AL: "Hydroxy and methoxy substituted N1,N4-diarylidene-S-methylthiosemicarbazon e iron(III) and nickel(II) complexes", TRANSITION METAL CHEMISTRY, KLUWER ACADEMIC PUBLISHERS, DO, vol. 29, no. 8, 1 November 2004 (2004-11-01), pages 880-884, XP019269113, ISSN: 1572-901X
- BELKIS ATASEVER ET AL: "Cytotoxic activities of new iron(III) and nickel(II) chelates of some S-methyl-thiosemicarbazones on K562 and ECV304 cells", INVESTIGATIONAL NEW DRUGS ; THE JOURNAL OF NEW ANTICANCER AGENTS, KLUWER ACADEMIC PUBLISHERS, BO, vol. 28, no. 4, 4 June 2009 (2009-06-04), pages 421-432, XP019816883, ISSN: 1573-0646
- BAL ET AL: "Synthesis, characterisation and cytotoxic properties of the N<1>,N<4>-diarylidene-S-methyl-thiosemicar bazone chelates with Fe(III) and Ni(II)", EUROPEAN JOURNAL OF MEDICINAL CHEMISTRY, EDITIONS SCIENTIFIQUE ELSEVIER, PARIS, FR, vol. 42, no. 2, 23 February 2007 (2007-02-23), pages 161-167, XP005901168, ISSN: 0223-5234, DOI: 10.1016/J.EJMECH.2006.09.004
- BAL-DEMIRCI T ET AL: "Iron(III) and nickel(ii) complexes as potential anticancer agents: Synthesis, physicochemical and structural properties, cytotoxic activity and DNA interactions", NEW JOURNAL OF CHEMISTRY 20150701 ROYAL SOCIETY OF CHEMISTRY GBR, vol. 39, no. 7, 1 July 2015 (2015-07-01), pages 5643-5653, ISSN: 1144-0546

## Description

### Technical Field of the Invention

The invention is related to new thiosemicarbazone -Fe chelate compounds displaying cytotoxic activity at low doses and selectively on leukaemia cells. The invention is further related to the production method of said compounds and special use thereof in the treatment of chronic leukaemia as an alternative or adjunct therapy agent.

### Background of the Invention

Chronic myeloid leukaemia (CML) is a type of leukaemia with slow progress and which usually does not suddenly worsen the condition of the patient. In chronic myeloid leukaemia, a genetic and non-inherent abnormality called Philadelphia chromosome (Bcr-Abl gene) is observed. The disease has 3 phases called chronic, accelerated and blastic phase (conversion to acute leukaemia). Patients are often in the chronic phase when the diagnosis is made. In chronic CML phase, it is aimed levelling down the blood cell number to normal and killing the cells carrying the Bcr-Abl gene. The aim in the accelerated or blast phase is to kill the cells carrying Bcr-Abl gene or to revert the disease to the chronic phase. The number of leucocytes in blood may be variable whereas in chronic leukaemia said number is increased. It is observed that even though busulfan and hydroxyurea, drugs used many years before, can make positive changes in the blood tests, no complete cure is obtained. Moreover, the use of these drugs is restricted because of their toxic effect on normal cells. The interferon discovered later on could decrease the Philadelphia chromosome and eliminate it in a small ratio of CML patients and by this means alpha interferon is started to be used in chronic phase. The medicine called Imatinib (Glivec), which is directed to eliminate the effect of Philadelphia chromosome having tyrosine kinase inhibitive activity is one of the most important first step medicines in CML patients. However, despite all these choices, the serious side effects of said used medicines and the fact that leukaemia cells develop resistance against them after a while and make them ineffective restrict their use and still no complete cure is provided. For this reason, research for new medicines continues and particularly the development of medicines having selective effect, which do not harm normal cells in the body at doses used, is crucial.

Recently, the antitumor effect of inorganic substances and partially metal complexes and the use thereof in cancer treatment is attracting attention *(*Debra C.Q., Kathy A. K. and Earl R. K., Antivir. Res., 71(1), 24-30, (2006*)).* Among these substances, thiosemicarbazones display therapeutic effect against bacteria and viral infections, tuberculosis, leprosy and cancer. Metisazone, one of the first antiviral drugs of thiosemicarbazone group is used in the prevention of smallpox.

Thiosemicarbazone, which is formed by the condensation of aldehydes or ketones with thiosemicarbazide compounds, constitutes an important class of ligand. It is observed that their biological activities change depending on the aldehyde or ketone group that they have *(*Cukurovali A.; Yilmaz I., Gur S., Kazaz C., Eur. J. Med. Chem., 41(2), 201-207, (2006*)).* The biological activity in such systems are correlated with the changes in the metal atom itself, the substitution reactions comprising more unstable extra planar ligands or substituents bound to ring system *(*Altun A., Kumru M., Dimoglo A., J. Mol. Struct, 535, 235 - 246 (2001*)).*

At the same time researches show that the biological activities of ligands increase by the metal complex formation *(*Saryan L.A., Ankel E., Krishnamurti C., Petering D.H., Elford H., J. Med. Chem., 22(10), 1218-21, (1979*);* LIBERTA A.E., West D.X., Bio. Metals., 5(2), 121-126, (1992*)).* The cytotoxic effects of thiosemicarbazones which are formed by metals such as iron, zinc, copper, nickel, palladium, platinum, ruthenium, on standard tumor cell lines are shown in various studies. The proliferation of cancer cells which are over proliferated is prevented by consuming the iron of said cells through the chelate effect of iron or nickel compounds of thiosemicarbazones. The fact that compounds of the same dose display less toxic effect on normal cells can be explained by their less need for iron. The mechanisms herein are still under investigation and are not clear but this selective effect on normal and cancer cells has therapeutic importance. Particularly, the reason for starting clinical studies (Phase I and II) in cancer therapy with a derivative of thiosemicarbazone triapine (3-aminopiridin-2-carboxyaldehyde thiosemicarbazone) which binds iron ions by giving chelates is that kind of selectivity *(*Richardson Des R., Sharpe P.C., Lovejoy D.B., Senaratne D., Kalinowski D.S., Islam M, and Bernhardt P.V., J. Med. Chem., 49(22), 6510-21, (2006*);* Whitnall M., Howard J., Ponka P., and Richardson D.R., PNAS, 103(40), 14901-14906, (2006*);* Danuta S.K., Philip C.S., Paul V. Bernhardt, and Des R. Richardson DR, J Med Chem, 50(24),6212-6225, (2007*)).* The following proteins can be important proteins which need iron for activity: cytochromes (proteins having Fe-S center), lipoxygenases, cyclooxygenases and ribonucleotide reductase. Iron plays an important role with regard to the increase of proteins at their active sites in a wide area comprising energy metabolism, respiration and DNA synthesis. Ribonucleotide reductase which is an enzyme comprising Fe is a requirement for the conversion of ribonucletides to deoxyribonucleotides in DNA synthesis. In the studies of Riccardson and colleagues, it is shown that the anticancer effect of Triapine (3-aminopiridin-2-carboxyaldehide thiosemicarbazone) synthesized is provided via the inhibition of ribonucleotide reductase enzyme which provides the conversion of ribonucletides to deoxyribonucleotides by impairing DNA synthesis.

It is reported that some thiosemicarbazones create damages by binding to DNA non-covalently with their aromatic groups. Another antitumor mechanism is the stabilization of DNA via the alkylation of thiol groups in TOPO II-DNA complex which leads to the apoptosis. 5-nitoimidazole thiosemicarbazone impairs DNA synthesis by damaging the purine and pyrimidine bases (Rodriguez-Arguelles M.C., López-Silva E.C., Sanmartín J., Bacchi A., Pelizzi C., Zani F., Inorg. Chim. Acta., 357, 2543-2552, (2004*);* Rodriguez-Arguelles MC, López-Silva EC, Sanmartín J, Pelagatti P, Zani, F; Inorg Biochem., 99(11), 2231-2239, (2005*)).*

In a study of the inventors, S-methyl-thiosemicarbazones of the 2-hydroxy-R-benzaldehyde reacted with corresponding aldehydes in the presence of FeCl₃ and NiCl₂ are disclosed whereby cytotoxicity of the said thiosemicarbazone compounds has been focus of this study *(*BELKIS ATASEVER ET AL: "Cytotoxic activities of new iron(llI) and nickel(ll) chelates of some S-methyl-thiosemicarbazones on K562 and ECV304 cells", INVESTIGATIONAL NEW DRUGS; THE JOURNAL OF NEW ANTICANCER AGENTS, vol. 28, no. 4, 2009, pages 421-432*).* This document, however is silent about apoptosis of the compounds for rendering them as suitable and feasible agents for use in treatment of cancer.

In the study of Ferrari and colleagues made in 3 different leukaemia cell lines (CEM, K562, U937), only in CEM and K562 cells the apoptosis is initiated with the copper complexes of 40 µg/ml 5-formyluracil thiosemicarbazone. This is a quite high dose. Again, in another study made in U937 cells with nickel complexes, IC50=14.4 µM even though it is comparatively a low, is still a very high dose (Buschini A, Pinelli S, Pellacani C, Giordani F, Belicchi Ferrari M, Bisceglie F, Giannetto M, Pelosi G, Tarasconi P , J Inorg Biochem 103(5):666-677 (2009*)).*

In cancers different than leukaemia ((MCF-7, TK-10, UACC-62)), within the studies made with the palladium complexes of benzolpyridine thiosemicarbazones, the effective cytotoxic concentration is found as 13.8 µM - 12.9 µM (Rebolledo A.P, Vieites M, Gambino D, Piro OE, Castellano EE, Zani CL, Souza-Fagundes EM, Teixeira LR, Batista AA, Beraldo H ; J Inorg Biochem 99(3):698-706 (2005*)*). In another study, the HCTs group [a-(N)-heterocyclic carboxaldehyde thiosemicarbazones] thiosemicarbazones L1210 are found cytotoxic in leukaemia cells at concentration of 4.2 - 1.3 µM (Li J, Zheng L.M., King I, Doyle TW, Chen S.H, Curr. Med. Chem. 8:121-133 (2001)).

Drugs and chemicals usually stimulate apoptosis at low doses and necrosis at high doses. Necrosis is an undesirable consequence because it stimulates inflammation and harms the surrounding tissues and cells. Most cancer drugs affect through creating DNA damage and drag the cancer cells to apoptotic process by benefiting the insufficiency in DNA repair mechanisms *(*Kaufmann S.H., Earnshaw W.C., Induction of apoptosis by cancer chemotherapy; Exp. Cell Res., 256(1), 42-9, (2000*)).* In order to increase the efficiency of the cancer drugs and to prevent the possible resistance mechanisms, it is the subject of current research to identify the apoptotic pathways activated by anticancer agents. Studies ongoing for many years show that most of the drugs used in cancer therapy can trigger apoptosis (Fischer U., and Schulze-Osthoff K., Pharmacol. Rev., 57(2), 187-215 (2005*)*)*.* Activation of apoptotic pathways are one of the main principles of cancer therapy. In another study, it is reported that the complexes of benzolpyridine thiosemicarbazones with antimonies cause DNA fragmentation at a rate of 60% in HL-60 cells (Reis D.C., Pinto M.C., Souza-Fagundes E.M., Wardell S.M., Wardell J.L., Beraldo H., Eur J Med Chem, 45(9):3904-10, (2010*)).* Again, after the incubation of thiosemicarbazones with palladium ([Pd(L2)(PPh3)]) within HL60 cells for 24 hours, the flow is performed and 27% of apoptotic and 4.5% of necrotic cells are found (Halder S., Peng S.M., Lee G.H., Chatterjee T., Mukherjee A., Dutta S., Sanyald U. and Bhattacharya S., New J. Chem., 32, 105-114, (2008*)).*

Notably, other metal complexes of the thiosemicarbazones known in the prior art are effective only in a narrow range of cancer cells and can display the sufficient cytotoxic effect only at high concentrations. In the prior art, as it would be appreciated by the person skilled in the art, high cytotoxic concentrations (IC₅₀) are not only effective on cancer cells but also do have the potential of destructive effect on other cells of the body. Furthermore, the triggering of necrosis in addition to apoptosis is another problem of the compounds of the prior art.

The inventors have realized the biological activities of Fe (III) complexes of N¹,N⁴-diariliden-S-metil-thiosemicarbazone obtained by template synthesis, having the ONNO coordination despite the fact that there are many studies on the biological activities of complexes in NS, DNS coordination mode of thiosemicarbazones. Moreover, it has been determined that iron chelates obtained in purified form are inefficient in endothelial cells at same doses and effectively cytotoxic in K562 leukemic cells.

Hence, one of the objectives of the current invention is to eliminate the problems related to the compounds having the high cytotoxicity concentrations (approximately 40 µg/ml) disclosed in the literature presented above (i.e. Ferrari and colleagues).

Another objective of the present invention is to present novel selective compounds which are only specific to cancer cells and yet not cytotoxic on endothelial cells.

Another objective of the current invention is to present novel compounds which provide cell death by apoptotic pathway which is more harmless instead of necrotic pathway which harms normal tissues.

Said objectives of the invention are achieved by new thiosemicarbazone -Fe and Ni chelate compounds disclosed in claim 1.

### Brief Description of the Figures

Figure 1 shows the immunocytochemical evaluation via H scoring of cytochrome C (A) and caspase 3 (B) activation in K562 cells incubated for 72 hours with thiosemicarbazones of the invention.
Figure 2 is the flow cytometry images of cells undergoing pre-apoptosis, post-apoptosis and necrosis in K562 cells dyed with Annexin V and PI after being incubated for 72 hours with thiosemicarbazones of the invention A) Control, B) I (0,6µg/ml), C) II (0,6µg/ml), D) III (0,6µg/ml), E) IV (0,1µg/ml).

### Detailed Description of the Invention

The invention presents new thiosemicarbazones compounds, particularly the Fe-complexes of these compounds, with the aim of providing alternative or support (combination therapy) to existing treatment options. The invention also comprises a method relating to the synthesis of these compounds in addition to the use of these in the manufacturing of drugs suitable for use in cancer treatment.

The investigation of antitumor effect of thiosemicarbazones and metal complexes thereof and their use in cancer therapy is becoming increasingly important and the elimination of existing problems (resistance, selectivity, treatment mechanism, etc.) is aimed.

As already disclosed in the prior art, despite the fact that the main thiosemicarbazone structures and various metal complexes thereof are known, it is also known that said compounds are still problematic and needed to be developed. The inventors have been investigated antitumor effects and mechanisms of action by producing a series of N¹, N⁴-diariliden-S-metil-thiosemicarbazone in the structure of a Fe- complex and discovered that the compounds of formula (I) presented below has surprisingly advantageous effects: 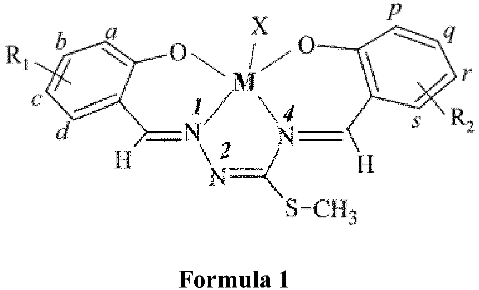
**Compound (I)** : M: Ni, X: -, R₁: 3-OH, R₂: 4-OCH₃
**Compound (II)** : M: Fe, X: Cl, R₁: 3-OH, R₂: 4-OCH₃
**Compound (III)** : M: Ni, X: -, R₁: 4-OH, R₂: 4-OCH₃
**Compound (IV)** : M: Fe, X: Cl, R₁: 4-OH, R₂: 4-OCH₃
whereas the present invention is solely directed to the Compound (IV).

IC₅₀ (the concentration inhibiting the proliferation of 50% of cells) of these 4 new compounds presented is identified by cytotoxicity tests in K562 (leukaemia) and ECV304 (endothelial) cells. Subsequently, it is studied to understand pathway through which the death of the cells is occurred (the mechanism of action) after the cells are treated for 72 hours at these concentrations. The apoptosis is analysed with immununocytochemical methods and flow cytometry and DNA fragmentation is analysed with diphenylamine reaction. The inventors have realized that besides that compounds I-IV have very low IC₅₀ concentrations, leukaemia cells die surprisingly at IC₅₀ concentrations applied whereas the endothelial cells require higher concentrations to die.

The table presented below shows the IC₅₀ values of K562 and ECV304 cells identified after being incubated for 72 hours with the compounds I-IV at various concentrations.

**Table 1**

| **Cells** | **IC₅₀ (microgram/ml)** | | | |
|---|---|---|---|---|
| | **I** | **II** | **III** | **IV** |
| **K562** | **0.61** | **0.62** | **0.65** | **0.1** |
| **ECV304** | **>5** | **>5** | **>5** | **>5** |

The inventors also analysed DNA fragmentation rates occurred in cells at the end of 72 hours at identified IC₅₀ concentrations. The results are given in Table 2.

**Table 2**

| **Cells** | **DNA fragmentation %** | | | | |
|---|---|---|---|---|---|
| | **Control** | **I** | **II** | **III** | **IV** |
| **K562** | **18±4.9** | **80±1.4** | **79±0.7** | **75±1.4** | **84±1.4** |
| **ECV304** | **15±1.2** | **20±0.5** | **18±2.1** | **22±1.7** | **28±2.4** |

The compounds of the invention show high efficiency at low concentrations at the level of IC₅₀ = 0.1 µg/ml in K562 type of cancer cells given as example. Considering the values at the level of IC₅₀ = 40 µg/ml obtained by Ferrari and his colleagues in the trials performed in cells of the same type and which are cited in the prior art, the advantages of the thiosemicarbazone compounds of the invention would be appreciated by a person skilled in the art. Moreover, as it can be seen in Table 2, the fact that the compound of the invention allows a restricted DNA fragmentation on ECV304 cells show that said compounds is quite selective.

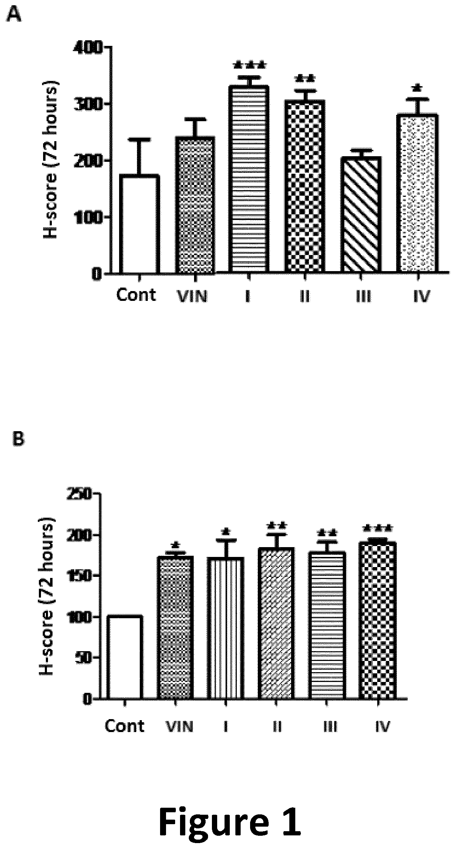

In the immunocytochemical evaluation presented in Figure 1, the cytochrome C (A) and caspase 3 (B) activation effects of thiosemicarbazone compounds on K562 cells after 72 hours of incubation is identified by H scoring. The thiosemicarbazone complexes subject to the current invention display statistically meaningful differences compared to control group (*p<0.05; ** p<0.01; *** p<0.001).

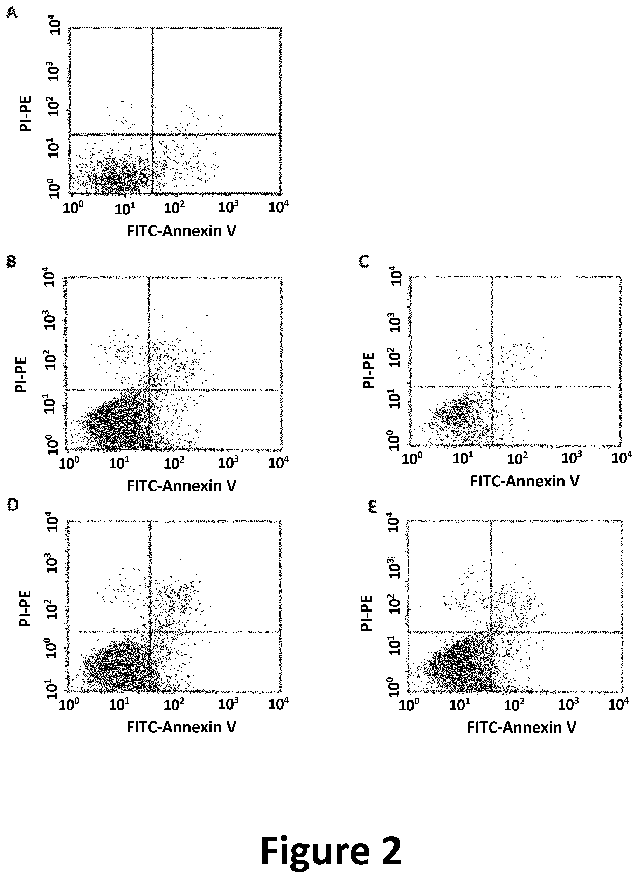

The Figure 2 gives the flow cytometry images of K562 cells, undergoing pre-apoptosis, post-apoptosis and necrosis, which are dyed with Annexin V and PI, after being incubated with thiosemicarbazones of the invention for 72 hours. Herein the images are as follows; A) Control, B) Compound I (0.6µg/ml), C) Compound II (0.6µg/ml), D) Compound (0.6µg/ml), and E) Compound IV (0.1µg/ml). The results found show that the compounds I-IV support apoptosis.

The inventors have characterized cells treated with compounds I-IV in the conditions of Figure 2 in order to better observe the mechanism of action of thiosemicarbazones presented in the current invention. The results are given in Table 3 below.

**Table 3**

| **Thiosemicarbazone derivatives** | **IC₅₀ (µg/ml)** | **Pre- apoptotic cell (%)** | **Post- apoptotic cell (%)** | **Necrotic cell (%)** | **Living cell (%)** | **Dead cell (%)** | **Apoptosis / Necrosis ratio** |
|---|---|---|---|---|---|---|---|
| **Control** | 0 | 12.59 | 18.86 | 0.78 | 67.82 | 32.18 | 16.14 |
| **I** | 0.6 | 34.85 | 27.39 | 2.57 | 35.18 | 64.82 | 13.56 |
| **II** | 0.6 | 30.9 | 18.54 | 3.15 | 47.4 | 52.6 | 9.80 |
| **III** | 0.6 | 37.5 | 23.23 | 3.17 | 36.11 | 63.89 | 11.82 |
| **IV** | 0.1 | 37.5 | 19.32 | 2.11 | 41.07 | 58.93 | 17.77 |

As it can be seen, the fact that necrotic cell rate is low and the high rate of apoptotic cells for all compounds shows that most of the dead cells are apoptotic and consequently the real mechanism responsible for the cell death triggered in cells is apoptosis.

As a consequence, in the scope of the experiments held by the inventors, in the immunohistochemical dye treatments performed for investigating apoptotic molecules (Figure 1) at 14^{th} and 72^{nd} hour, the fact that cas 3 and cyt C levels are higher significantly compared to controls show that the compounds of the invention provide apoptosis. DNA fragmentation is found high in all experiment groups (70-80%). These rates are compatible with IC₅₀ obtained with MTT test because IC₅₀ is the dose which kills the 50% of the cells. The fact that DNA fragmentation is a little higher suggests that the lower doses than those used herein can activate apoptosis. Furthermore, this rate is compatible with the percentage of total dead cells (52-64 %) which is the addition of apoptotic and necrotic cells obtained by annexin/PI dyeing.

As it can be understood from the data above, the compound IV of the invention among other thiosemicarbazone compounds displays additional special effects. This compound providing high efficiency with low IC₅₀ value (Table 1) is also advantageous since it allows cells at low rate. Because of the reasons presented, the Compound IV among the thiosemicarbazone compounds constitute the subject matter of the current invention. Accordingly, the compound shown with formula I is;
M: Fe,
X: Cl,
R₁: 4-OH, and
R₂: 4-OCH₃

In an exemplary production method of the compounds presented above, first a metal salt solution of FeCl₃ or NiCl₂ is prepared and triethyl orthoformic ester or triethyl orthoformate is added into said solution. Then a ligand solution containing 3-hydroxysalicylaldehyde-S-methylisothiosemicarbazone or 4-hydroxysalicyl aldehyde-S-methylisothiosemicarbazone with 4-methoxysalycylaldehyde is prepared and this solution is added into the FeCl₃ or NiCl₂. In the end of the method, the compounds shown with formula I are obtained and isolated.

The compounds (IV) shown with formula I can be suitable for use in cancer treatment. Said cancer is preferably leukaemia and more preferably is Chronic Myeloid Leukemia (CML).

The compounds used in the experimental data above are produced according to the procedures presented below.

### Example 1. Production of N(1)-3-hydroxysalicylidene-N(4)-4-methoxysalicylideneisothio-semicarbazidato Nickel (II):

To prepare the NiCl₂ solution, NiCl₂.6H₂O (1 mmol) is dissolved in 30 ml ethanol. It is waited for 1 hour after the addition of triethyl orthoformic ester. Ligand solution is prepared by dissolving 3-hydroxysalicylaldehyde-S-methylisothiosemicarbazone (1 mmol) with 4-methoxysalycylaldehyde (1 mmol) in 100 ml ethanol. Ligand solution is added into the metal salt solution dropwise. The product precipitated by the addition of triethylamine is filtered. The red colored complex compound is washed with ethanol-ether mixture (1/1): Template product is separated with column chromatography. The purity of the product is controlled with layer chromatography. It is dried in vacuum with P₂O₅ for 12 hours. Yield: 42%.

### Example 2. Production of Monochloro-N(1)-3--hydroxysalicylidene-N(4)-4-methox salicylideneisothiosemicarbazidato Iron (III):

Metal solution is prepared by dissolving FeCl₃.6H₂O (1 mmol) in 30 ml ethanol and adding triethyl orthoformic ester after being waited for 18 hours. For the ligand solution 3-hydroxysalicylaldehyde-S-methylisothiosemicarbazone (1 mmol) and 4-methoxysalycyl aldehyde (1 mmol) were taken. Both compounds were dissolved in 100 ml ethanol. Ligand solution is added into the FeCl₃ solution dropwise and is refluxed. 2 hours later, shiny black colored crystals precipitated are filtrated. They are washed with ethanol-ether mixture (1/1). The template product is separated with column chromatography. The purity thereof is controlled with the thin-layer chromatography. It is dried in vacuum with P₂O₅ for 12 hours. Yield: 16 %.

### Example 3. Production of N(1)-4-hydroxysalicylidene-N(4)-4-methoxysalicylideneisothio-semicarbazidato Nickel (II):

To prepare the NiCl₂ solution, NiCl₂.6H₂O (1 mmol) is dissolved in 30 ml ethanol. It is waited for 2 hours after the addition of triethyl orthoformic ester. Ligand solution is prepared by dissolving 4-hydroxysalicylaldehyde-S-methylisothiosemicarbazone (1 mmol) with 4-methoxysalycylaldehyde (1 mmol) in 100 ml ethanol. Ligand solution is added into the metal salt solution dropwise. The product precipitated by the addition of triethylamine is filtered. The red colored complex compound is washed with ethanol-ether mixture (1/1): Template product is separated with column chromatography. The purity of the product is controlled with layer chromatography. It is dried in vacuum with P₂O₅ for 12 hours. Yield: 28%.

### Example 4. Production of Monochloro-N(1)-4-hydroxysalicylidene-N(4)-4-methoxy-salicylideneisothiosemicarbazidato Iron (III):

Metal solution is prepared by dissolving FeCl₃.6H₂O (1 mmol) in 20 ml ethanol and adding triethyl orthoformic ester after being waited for 11 hours. For the ligand solution 4hydroxysalicylaldehydeSmethylisothiosemicarbazone (1 mmol) and 4-methoxysalycyl aldehyde (1 mmol) were taken. Both compounds were dissolved in 100 ml ethanol. Ligand solution is added into the FeCl₃ solution dropwise and is refluxed. 4 hours later, shiny black colored crystals precipitated are filtrated. They are washed with ethanol-ether mixture (1/1). The template product is separated with column chromatography. The purity thereof is controlled with the thin-layer chromatography. It is dried in vacuum with P₂O₅ for 12 hours. Yield: 12 %.

All of the complexes (I-IV) produced herein are synthesized with methods above and characterized. The substituent distribution of 4 complexes are shown in Table 1.

In the table, the analytical and spectral data of the compounds are shown in the following order: colour, yield (%), melting point (°C), molar conductivity (ohm⁻¹cm⁻²mol⁻¹, in 10³ M DMSO, 25 °C), µ_{eff} (BM), elemental analysis, FT-IR (KBr, cm⁻¹) and ¹H-NMR (for Ni complexes) (DMSO-d₆, 25°C, ppm), Mass (for Fe complexes).

**Table 4. Characterization of the Complexes**

| | | |
|---|---|---|
| **I** | Ni/3-OH/4-OCH₃ | Red, 42; 276-277(decomposition); 5.6; 0.06; Anal. Calculated: |
| | | C₁₇H₁₅N₃O₄SNi (415,7 g): C, 49.07; H, 3.63; N,10.10; S, 7.71, Observed: C, 49.12; H, 3.63; N, 10.05; S, 7.70%. IR (cm⁻¹): v(OH) 3437, v(C=N) 1609, 1597,1580 v(C-O) 1150, 1108. ¹H-NMR: δ 8.45 (s, 1H, OH), 8.19 (s, 1H, CH=N¹), 8.14 (s, 1H, CH=N⁴), 6.42 (dd, J=9.27, J=2.44, 1H, b), 6.50 (t, J=7.81, 1H, c), 7.65 (dd, J=9.27, 1H, d), 6.47 (d, J=2.44, 1H, p), 6.78 (dd, J=7.32, J=1.46, 1H, r), 7.01 (dd, J=7.80, J=1.47, 1H, s), 3.81 (s, 3H, O-CH₃), 2.70 (s, 3H, S-CH₃). |
| **II** | Fe/3-OH/4-OCH₃ | Shiny Black, 16, >399, 20.3; 5.82; Anal. Calculated: |
| | | C₁₇H₁₅N₃O₄SFeCl (448.35 g/mol-g.): C, 45.50; H, 3.34; N, 9.37; S, 7.14; Observed: C, 45.44; H, 3.59; N, 9.45; S, 7.10%. FT-IR (KBr, cm⁻¹) (C=N) 1612, 1601, 1578, (C-O)ₐᵣₒₘ 1158,1131, Mass : m/z (-c APCI): 448 (M⁺, 100.00), 449 (MH⁺, 21.35), 447 (M⁺-H, 4.04), 446 (M⁺-2H, 8.16), (+c APCI): 413 (M⁺ ,-Cl, 100.00), 414 (MH⁺ ,-Cl, 20.38) |
| **III** | Ni/4-OH/4-OCH₃ | Red, 28; 276-277(decomposition); 7.9; 0.18; Anal. Calculated: |
| | | C₁₇H₁₅N₃O₄SNi (415,7 g): C, 49.07; H, 3.63; N,10.10; S, 7.71, Observed: C, 49.04; H, 3.65; N, 10.02; S, 7.67%. IR (cm⁻¹): v(OH) 3437, v(C=N) 1608, 1598, 1582 v(C-O) 1150, 1108. ¹H-NMR: δ 10.08 (s, 1H, OH), 8.22 (s, 1H, CH=N¹), 8.03 (s, 1H, CH=N 4), 6.23 (d, J=2.29, 1H, a), 6.21 (dd, J=8.24, J=2.29, 1H, c), 7.61 (d, J=9.61, 1H, d), 6.46 (d, J=2.29, 1H, p), 6.38 (dd, J=9.15, J=2.28, 1H, r), 7.34 (d, J=8.69, 1H, s), 3.80 (s, 3H, O-CH₃), 2.66 (s, 3H, S-CH₃). |
| **IV** | Fe 4-OH/4-OCH₃ | Black, 12; >390; 7.9; 0.18; Anal. Calculated: |
| | | C₁₇H₁₅N₃O₄SFeCl (448.35 g/mol-g.): C, 45.50; H, 3.34; N, 9.37; S, 7.14; Observed: C, 45.59; H, 3.35; N, 9.42; S, 7.39%. IR (cm⁻¹): v(OH) 3440, v(C=N) 1608, 1597, 1582 v(C-O) 1152, 1106. Mass : m/z (-c APCI): 448 (M⁺, 100.00), 449 (MH⁺, 22.86), 447 (M⁺-H, 4.15), 446 (M⁺-2H, 8.98), (+c APCI): 413 (M⁺ ,-Cl, 100.00), 414 (MH⁺ ,-Cl, 22.23) |

## Claims

1. A compound having the formula (I) 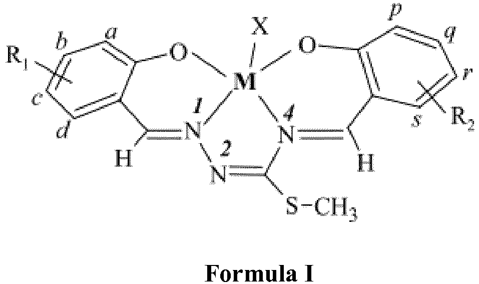 wherein;
M: Fe, X: Cl, R₁: 4-OH, and R₂: 4-OCH₃
for use in treatment of cancer.

2. The compound for use according to claim 1, wherein said cancer is leukaemia.

3. The compound for use according to claim 2, wherein said leukaemia is Chronic Myeloid Leukemia (CLM).

## Patentansprüche

1. Eine Verbindung mit folgender Formel (I): 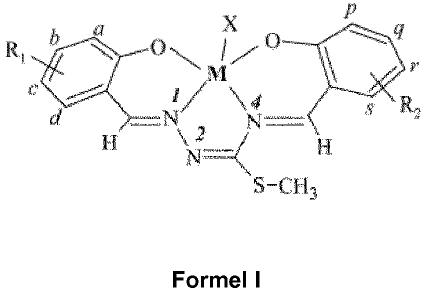 wobei folgendes gilt:
M: Fe, X: CL, R₁: 4-OH und R₂: 4-OCH₃
zur Verwendung bei der Behandlung von Krebs.

2. Die Verbindung zur Verwendung gemäß Anspruch 1, bei der der Krebs Leukämie ist.

3. Die Verbindung zur Verwendung gemäß Anspruch 2, bei der die Leukämie chronische myeloische Leukämie (CLM) ist.

## Revendications

1. Composé ayant la formule (I) : 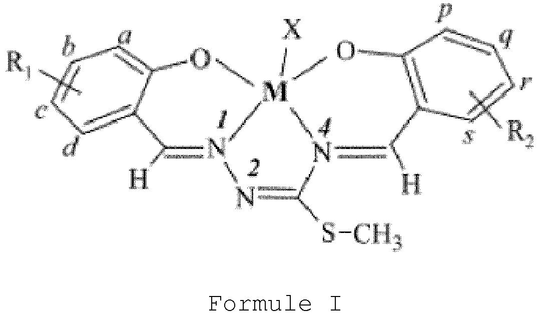 dans laquelle :
M : Fe, X : Cl, R₁ : 4-OH et R₂ : 4-OCH₃
pour une utilisation dans le traitement du cancer.

2. Composé pour une utilisation selon la revendication 1, dans lequel ledit cancer est une leucémie.

3. Composé pour une utilisation selon la revendication 2, dans lequel ladite leucémie est la leucémie myéloïde chronique (LMC).
